# EUROPÄISCHE PATENTSCHRIFT

(11) **EP 0 441 740 B1**
(45) Veröffentlichungstag und Bekanntmachung des Hinweises auf die Patenterteilung: **22.09.1993**
(21) Anmeldenummer: 91810049.6
(22) Anmeldetag: 22.01.1991
(51) Int. Cl.: B29B 9/08, A61J 3/02, G01F 11/18

(54) **Verfahren und Vorrichtung zur Dosierung eines feinkörnigen Pulvers**
Method and device for dosing a fine particles powder
Procédé et dispositif pour le dosage d'une poudre à grains fins

(30) Priorität: 29.01.1990 CH 266/90
(43) Veröffentlichungstag der Anmeldung: 14.08.1991
(73) Patentinhaber: CIBA-GEIGY AG, 4002 Basel (CH)
(72) Erfinder: Boesch, Beate, F-68740 Roggenhouse (FR); Khanna, Satish Chandra, Dr., CH-4103 Bottmingen (CH)

(56) Entgegenhaltungen:
- DE-A- 3 332 528
- FR-A- 2 140 180
- US-A- 4 709 837
- PHARMAZIE, Band 35, Nr. 4, April 1980, Seiten 237-249; M. DITTGEN et al.: "Zurpharmazeutischen Technologie der Granulierung"

## Beschreibung

Die Erfindung betrifft ein Verfahren zur Dosierung kleinster Mengen eines nicht oder schlecht fliessfähigen Pulvers gemäss Oberbegriff von Patentanspruch 1 und eine entsprechende Vorrichtung gemäss Oberbegriff von Patentanspruch 7.

Viele feinkörnige Pulver zeichnen sich unter anderem dadurch aus, dass sie entweder schlecht oder gar nicht fliessfähig sind, auf gar keinen Fall jedoch regelmässig fliessen. Durch das schlechte bzw. sehr unregelmässige Fliessen des Pulvers bedingt ergeben sich bei der exakten Dosierung insbesondere sehr kleiner Mengen eines solchen Pulvers erhebliche Schwierigkeiten. Oft fliesst das Pulver beim Einfüllen in ein Dosierbehältnis bzw. in eine Dosierkammer nur schubweise in die Dosierkammer, weil die Pulverkörner entweder aneinander oder an der Oberfläche einer Zuführeinrichtung kleben bleiben. Fülltrichter zum Einfüllen des Pulvers in die Dosierkammer verstopfen so sehr leicht. Das bedeutet, dass wenn die Dosierkammer in regelmässigen Zeitabständen entleert wird, die in ihr befindliche Pulvermenge sehr grossen Schwankungen unterliegen kann. Solange die Pulverzufuhr aufgrund des Verklebens sehr gering ist, gelangt während der Zeit, in der die Dosierkammer gefüllt werden soll, auch nur eine geringe Pulvermenge in die Dosierkammer. Andererseits, wenn dann ein solcher Pulverschub dem Dosierbehältnis zugeführt wird, im Falle des verstopften Fülltichters also die Verstopfung sich löst, läuft das Dosierbehältnis über, so dass ein entsprechendes Ueberlaufbehältnis vorgesehen werden muss. Bei Dosierverfahren, bei denen es ganz besonders darauf ankommt, dass die dosierte Pulvermenge stets konstant ist bzw. nur sehr kleine relative Gewichtsschwankungen der dosierten Pulvermenge zulässig sind, beispielsweise bei der Dosierung pharmazeutischer Pulver, ist diese schlechte Fliesseigenschaft des Pulvers also besonders hinderlich. Im Hinblick auf eine spätere Verabreichung der dosierten Pulvermenge ist es hier nämlich unabdingbar, dass der Dosierkammer stets die gleiche Pulvermenge entnommen wird. Das heisst, dass unter Umständen Füllstandsanzeiger für die Dosierkammer erforderlich werden, um zu gewährleisten, dass der Dosierkammer stets die gleiche Pulvermenge entnommen wird, was einen nicht unerheblichen Aufwand bedeutet.

Eine Möglichkeit, das schlecht oder gar nicht fliessfähige Pulver fliessfähig zu machen und so die Dosierung zu vereinfachen, besteht darin, dem Pulver ein sogenanntes " Schmiermittel" zuzusetzen. Solche Schmiermittel bilden durch Adhäsionskräfte zwischen der Oberfläche der feinen Pulverkörner und der Oberfläche des Schmiermittels fliessfähige Pulver - Schmiermittel - Konglomerate. Dieses Vorgehen ist aber in zweierlei Hinsicht nachteilig. Zum einen können beispielsweise bei Therapeutika zur Behandlung der menschlichen Lunge, die auf diese Weise hergestellt werden, die Schmiermittel vom "Einwegsystem" Lunge vielfach nicht abgebaut werden, zum anderen sind die auf diese Weise gebildeten Konglomerate zu grob, um eine ausreichend exakte Dosierung sehr kleiner Mengen zuzulassen. Ein Beispiel für ein solches von der Lunge nicht abbaubares Schmiermittel ist Stearinsäure (feste Phase). Bei der Benutzung von flüssigen Schmiermitteln zur Bildung der Konglomerate gibt es zwar Trockenverfahren, mit deren Hilfe die Schmiermittel nach der Konglomeratbildung wieder aus den Konglomeraten entfernt werden können. Die getrockneten Konglomerate zerfallen aber dann sofort wieder zu nicht fliessfähigem feinem Pulver oder die Konglomerate sind sehr fest, je nachdem, welches Trockenverfahren angewendet wird; im erstgenannten Fall liegen nach dem Trocknen wieder die feinen schlecht fliessfähigen Pulverkörner zum Weitertransport vor bzw. im zweiten Fall ist eine Verkleinerung der Konglomerate nicht mehr möglich, was aber hinsichtlich der beabsichtigten Verabreichung (z.B. Pulverinhalation) unerlässlich ist, da die Konglomerate in unzerkleinertem Zustand zu grob sind. Ausserem wurde auch schon beobachtet, dass sich beim Zusatz von flüssigen Schmiermitteln die Grösse der Kristalle der einzelnen feinen Pulverkörner verändert, so dass selbst wenn eine Zerkleinerung der Konglomerate nach der Trocknung möglich wäre, die Pulverkörner für eine Inhalation noch zu grob wären.

Diese Probleme werden durch das erfindungsgemässe Verfahren, wie im Patentanspruch 1 definiert, und durch die entsprechende Vorrichtung, wie im Patentanspruch 7 definiert, gelöst. Besonders vorteilhafte Ausgestaltungen des Verfahrens bzw. der Vorrichtung ergeben sich aus den jeweils abhängigen Ansprüchen.

Im folgenden wird die Erfindung anhand der Zeichnung näher erläutert; es zeigen:
- Fig. 1: eine schematische Uebersichtsdarstellung der Vorrichtung zur Durchführung des erfindungsgemässen Verfahrens, teilweise im Axialschnitt
- Fig. 2a-c: eine Darstellung der nicht oder schlecht fliessfähigen Pulverkörner und der fliessfähigen Körneragglomerate

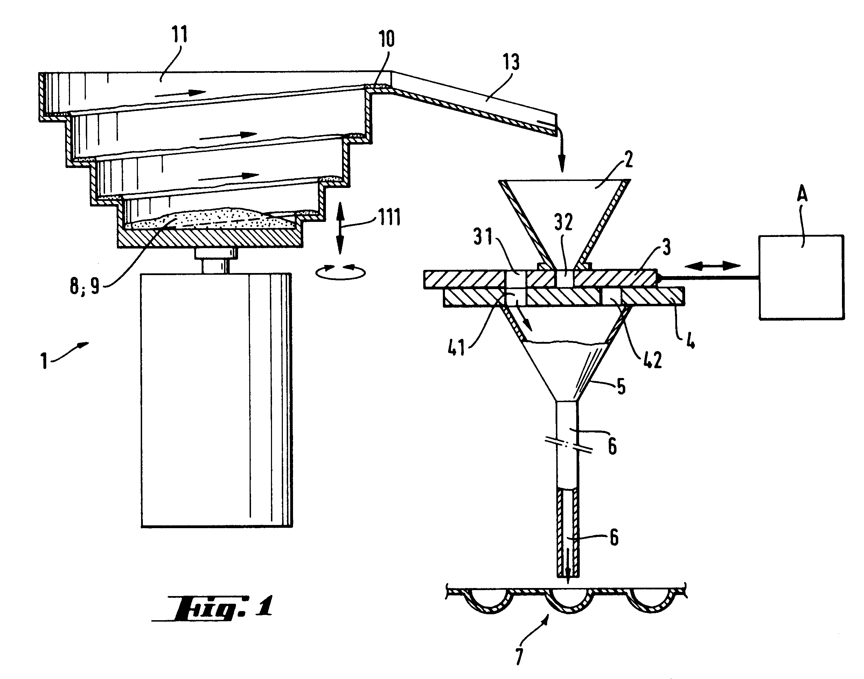

Die in Fig. 1 dargestellte Vorrichtung umfasst einen Vibrationsförderer 1, einen ersten Fülltrichter 2, Dosiermittel in Form einer bewegbaren ersten Platte 3 mit zwei Oeffnungen 31 und 32 und einer ortsfesten zweiten Platte 4 mit zwei Oeffnungen 41 und 42, einen zweiten Fülltrichter 5 sowie ein Rohr 6.

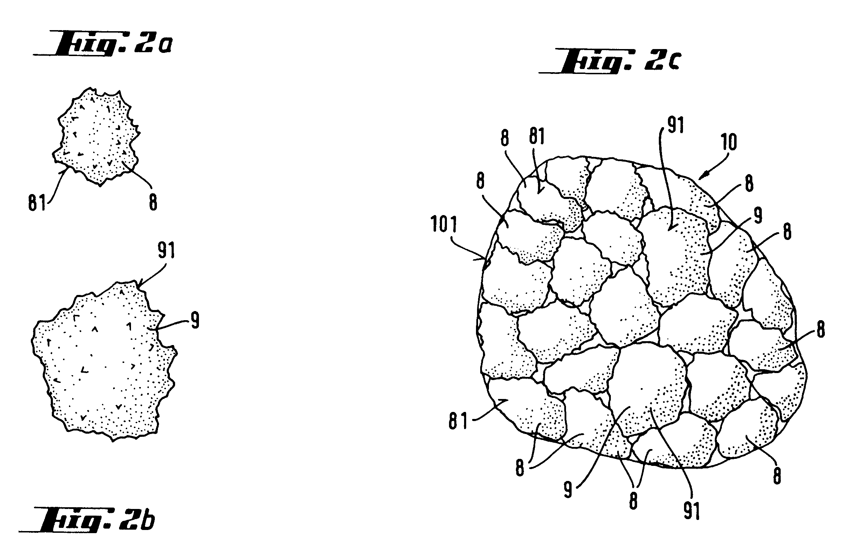

Das nicht oder schlecht fliessfähige Pulver 8 bzw. 9 wird in das Zuführbehältnis 11 des Vibrationsförderers 1 eingefüllt. Während man normalerweise erwartet, dass durch mechanische Vibrationen dieses Zuführbehältnisses 11 vorhandene gröbere Körner in feinere Körner zerteilt werden, passiert hier genau das Gegenteil. Durch die Vibrationen des Zuführbehältnisses 11 in Richtung der Pfeile 111 werden die feinen Körner des Pulvers 8 bzw. 9 zu fliessfähigen, regelmässigen Körneragglomeraten 10 zusammengeballt (Fig.2). Die Agglomeration findet dabei ohne den Zusatz von Schmiermitteln wie Flüssigkeiten etc. statt. Die so gebildeten Körneragglomerate 10 ("Pellets") fliessen das wendeltreppenartig gewundene Zuführbehältnis 11 hinauf und über den Förderteil 13 dieses Behältnisses 11 in den ersten Fülltrichter 2. Der Vibrationsförderer 1 übernimmt damit auch die Funktion der Fördermittel, die das Pulver in Form der Pellets 10 den Dosiermitteln zuführen. Die Dichte des relativ gleichmässigen Körneragglomeratestromes, der in den Fülltrichter 2 hineinfliesst, hängt dabei von der Amplitude der mechanischen Vibrationen des Zuführbehältnisses 11 ab: Je grösser die Amplitude der Vibrationen ist, desto dichter ist der Körneragglomeratestrom in den Fülltrichter 2 hinein. Der Fülltrichter 2 führt diese Pellets dann direkt den Dosiermitteln zu.

Die Dosiermittel umfassen zwei relativ zueinander bewegbare Platten 3 und 4 mit jeweils zwei durchgehenden Oeffnungen 31 und 32 bzw. 41 und 42 sowie eine Automatik A zum Bewegen der bewegbaren oberen Platte 3. Die Automatik A bewegt diese Platte 3 zwischen zwei Relativstellungen, in denen die Platte 3 jeweils stehen bleibt, in regelmässigen Zeitintervallen hin und her. In der ersten Relativstellung, die hier dargestellt ist, ist die eine Oeffnung 32 der bewegbaren oberen Platte 3 von der ortsfesten unteren Platte 4 nach unten verschlossen und bildet so eine Dosierkammer. Gleichzeitig fluchtet diese Oeffnung 32 mit der Auslassöffnung 21 des ersten Fülltrichters 2, so dass die Dosierkammer 32 von oben mit Pellets 10 befüllt werden kann. Die andere Oeffnung 31 der bewegbaren oberen Platte 3 fluchtet in dieser ersten Relativstellung der beiden Platten 3 und 4 mit der Oeffnung 41 der ortsfesten unteren Platte 4 und ist somit nach unten geöffnet. Nach Ablauf des Intervalls bewegt die Automatik A die obere Platte 3 in die zweite Relativstellung. Das Intervall zwischen dem Hin- und Herbewegen der oberen Platte 3 ist dabei so bemessen, dass die gleiche Menge Pellets, die während dieses Intervalls in die Dosierkammer 32 eingefüllt worden ist, inzwischen über den Förderteil 13 des Vibrationsförderers 1 wieder in den ersten Fülltrichter 2 nachgeführt worden ist. Die in der Dosierkammer 32 befindlichen Pellets werden in der Dosierkammer 32 in die zweite Relativstellung mitbewegt, ohne zerdrückt oder zerquetscht zu werden. In dieser zweiten Relativstellung fluchtet die Oeffnung 32 der oberen Platte 3 mit der Oeffnung 42 der unteren Platte 4. Dadurch ist die Dosierkammer 32 geöffnet und die Pellets können durch die Oeffnung 42 der unteren Platte hindurch weiter zu den Abfüllmitteln gelangen. Die andere Oeffnung 31 der oberen Platte 3 ist in dieser zweiten Relativstellung von der unteren Platte 4 nach unten verschlossen und bildet so eine Dosierkammer. Gleichzeitig fluchtet sie nach oben mit der Auslassöffnung 21 des ersten Fülltrichters 2, so dass in dieser zweiten Relativstellung während der Entleerung der Dosierkammer 32 durch die Oeffnung 42 der unteren Platte 4 hindurch die Dosierkammer 31 mit Pellets gefüllt wird. Die untere Platte 4 mit ihren beiden Oeffnungen 41 und 42 fungiert also als Auslassventil. In jeder der beiden Relativstellungen verschliesst sie die Dosierkammer 31 bzw. 32, die gerade von oben befüllt wird. Zur gleichen Zeit wird die andere Dosierkammer 32 bzw. 31 von der entsprechenden Oeffnung 42 bzw. 41 der unteren Platte geöffnet und die dosierte Menge Pellets kann durch diese Oeffnung 42 bzw. 41 hindurch zu den Abfüllmitteln gelangen.

Die Abfüllmittel umfassen einen zweiten Fülltrichter 5, der in ein Rohr 6 mündet. Dieser zweite Fülltrichter 5 ist mit der ortsfesten unteren Platte 4 baulich verbunden und so angeordnet, dass er die durch die jeweilige Oeffnung 41 bzw. 42 der unteren Platte 4 hindurchtretende Pulvermenge auffängt. Die vom Fülltrichter 5 aufgefangene Pulvermenge wird von diesem in das Rohr 6 geführt, welches diese Pulvermenge einem dafür vorgesehenes Aufnahmegefäss 7 zuführt.

Als ein besonders geeigneter Vibrationsförderer 1 für die oben beschriebene Vorrichtung hat sich ein Wendelvibrator vom Typ WV 150 der Firma AFAG herausgestellt. Selbstverständlich eignet sich aber auch eine Vorrichtung mit einer Vibrationseinrichtung, die andersartig ausgebildet ist als der hier beschriebene Vibrationsförderer 1. Auch müssen die Fördermittel 13, die die Pellets 10 zu den Dosiermitteln transportieren, nicht ein Bestandteil des Vibrationsförderers 1 sein; sie können ebenfalls als eine selbständige Einheit ausgeführt sein. Es muss nur gewährleistet sein, dass die Pellets durch den Transport nicht wieder zerquetscht werden.

Die Dosierkammer 32 muss auch nicht notwendigerweise vollständig gefüllt sein, wenn die Automatik A die erste Platte 3 in die zweite Relativstellung bewegt, in der die Dosierkammer 32 aufgrund des Fluchtens der Oeffnung 32 der ersten Platte 3 und der Oeffnung 42 der zweiten Platte 4 entleert wird. Es muss nur gewährleistet sein, dass der Dosierkammer 32 stets die gleiche Menge Pellets entnommen wird ( Analoges gilt natürlich für die Dosierkammer 31 ). Wird die Dosierkammer 32 nicht vollständig gefüllt, so werden unter Umständen Füllstandsanzeiger erforderlich, die beim Erreichen der zu dosierenden Menge Pellets 10 an die Automatik A ein Signal geben, damit die Platte 3 in die andere Relativstellung bewegt wird. Das bedeutet zwar einen höheren Aufwand, hat aber gegenüber bekannten Vorrichtungen bzw. Verfahren immer noch den Vorteil, dass aufgrund der regelmässigen Zufuhr der Pellets 10 in die Dosierkammer 31 bzw.32 und aufgrund des kleinen Durchmessers der Pellets 10 eine exakte Dosierung auch sehr kleiner Pulvermengen möglich ist.

Weiterhin ist prinzipiell auch nur jeweils eine Oeffnung in jeder der Platten 3 und 4 erforderlich, um das Funktionieren der Vorrichtung zu gewährleisten. Beispielsweise können die Oeffnung 31 der ersten Platte 3 und die Oeffnung 41 der zweiten Platte 4 weggelassen werden. Wichtig ist, dass das Zeitintervall zwischen dem Bewegen der ersten Platte 3 in die jeweils andere Relativstellung so abgestimmt ist, dass in dem Zeitintervall, in dem die Dosierkammer 32 durch die Oeffnung 42 der zweiten Platte 4 hindurch entleert wird, die gleiche Menge Pellets 10 wieder dem Fülltrichter 2 zugeführt wird, die bei der Entleerung der Dosierkammer 32 entnommen wurde. Es kann auch eine Regelung vorgesehen sein, die aufgrund der Dichte des Stromes von Pellets 10 in die Dosierkammer 32 bzw. 31 die Automatik A so steuert, dass diese Bedingung erfüllt ist.

Des weiteren müssen auch der zweite Fülltrichter 5 und das Rohr 6 nicht baulich miteinander verbunden sein. Sie können auch jeweils als für sich selbständige Einheit ausgeführt sein. Besonders praktisch ist eine Vorrichtung, bei der der erste Fülltrichter 2, die beiden Platten 3 und 4, die Automatik A, der zweite Fülltrichter 5 sowie das Rohr 6, also die Dosiermittel und die Abfüllmittel, zu einer baulichen Einheit zusammengefügt sind.

Als Aufnahmegefäss 7 für das vom Rohr 6 transportierte Pulver kann beispielsweise eine handelsübliche pharmazeutische Kapsel oder eine in der pharmazeutischen Industrie allgemein übliche Blisterpackung dienen. Ebenso kommen natürlich auch in der pharmazeutischen Industrie übliche Aufbewahrungsflaschen für ein solches Aufnahmegefäss in Frage.

Das oben beschriebene Verfahren bzw. die oben beschriebene Vorrichtung ist besonders geeignet für die Dosierung sehr kleiner Mengen einer schlecht fliessfähigen Pulvermischung aus zuvor gemahlener und/oder gesichteter Lactose (Milchzucker) und Formoterol, hier in Form des Salzes Formoterol fumarat, dessen Bezeichnung nach der IUPAC-Nomenklatur "(±)-2'-Hydroxy-5'-[(RS)-1-hydroxy-2-[[(RS)-p-methoxy-α-methylphenethyl]-amino]ethyl]formanilid·fumarat#CDT#dihydrat" lautet. Formoterol ist ein Wirkstoff, der zur Behandlung von Erkrankungen der menschlichen Lunge bzw. der Atemwege eingesetzt wird, beispielsweise bei Asthmaerkrankungen. Der Wirkstoff wird mit Lactose zu einer Pulvermischung vermengt und die Pulvermischung inhaliert. Das Mischungsverhältnis Formoterol zu Gesamtmischung ( Formoterol + Lactose ) liegt dabei im Bereich von 1:10 bis 1:500 . Die mittlere Korngrösse des Formoterols beträgt etwa 5µm, die mittlere Korngrösse der Lactose liegt im Bereich kleiner 50µm, vorzugsweise beträgt sie 1µm bis 10µm. Die Figuren 2a-c zeigen, stark vergrössert, solche Formoterolkörner 8 (Fig. 2a) und Lactosekörner 9 (Fig. 2b), deren Oberflächen 81 bzw. 91 zackig und kantig sind, worin auch eine der Hauptursachen für die schlechte Fliessfähigkeit des Pulvers im nicht agglomerierten Zustand liegt. Im Unterschied dazu ist die Oberfläche 101 der durch die Vibrationen des Vibrationsförderers 1 gebildeten Pellets 10 (Fig. 2c) im wesentlichen abgerundet, so dass die Pellets 10 demzufolge besser fliessen können. Der mittlere Durchmesser dieser Pellets 10 liegt im Bereich von etwa 50 µm bis 2000 µm, je nachdem, welche mittlere Korngrösse die verwendete Lactose aufweist, die ja vor der Agglomeration gesichtet wird. Je grösser der mittlere Durchmesser der Lactosekörner 9 ist, desto weicher und instabiler werden auch die Agglomerate. Besonders stabile und fliessfähige Pellets 10 ergeben sich bei der Verwendung von Formoterol 8 mit einem mittleren Durchmesser von 5 µm und Lactose mit einem mittleren Durchmesser von 10 µm, wobei das Mischungsverhältnis Formoterol zu Gesamtmischung (Formoterol+Lactose) ca. 1:40 beträgt. Die Frequenz der Vibrationen, die die Bildung der regelmässigen Pellets ermöglichen, liegt dabei vorzugsweise im Bereich um 100 Hz. Selbstverständlich sind auch andere Vibrationsfrequenzen möglich.

Wie bereits weiter oben erwähnt, eignet sich das oben beschriebene Verfahren bzw. die oben beschriebene Vorrichtung für die exakte Dosierung sehr kleiner Mengen nicht oder schlecht fliessfähiger Pulver. Ein Einsatzgebiet, für das sich das Verfahren bzw. die Vorrichtung besonders eignet, ist die exakte Dosierung pharmazeutischer Pulver, insbesondere einer Mischung aus Formoterol und Lactose, wo allenfalls sehr kleine relative Gewichtsschwankungen zulässig sind und zur Bildung von Agglomerationen keine Schmiermittel (z.B. Flüssigkeiten) zugesetzt werden können.

## Patentansprüche

1. Verfahren zur Dosierung kleinster Mengen eines nicht oder schlecht fliessfähigen feinkörnigen Pulvers (8,9), bei dem das Pulver einer Dosierkammer (31,32) zugeführt und diese regelmässig entleert wird, dadurch gekennzeichnet, dass vor der Pulverzufuhr in die Dosierkammer (31,32) die nicht oder schlecht fliessfähigen Körner (8,9) des Pulvers durch mechanische Vibrationen zu fliessfähigen Körneragglomeraten (10) zusammengeballt werden.

2. Verfahren nach Anspruch 1, dadurch gekennzeichnet, dass zur Zusammenballung zu fliessfähigen Körneragglomeraten ( 10) und zur anschliessenden Zufuhr dieser Agglomerate (10) in die Dosierkammer (31,32) ein Vibrationsförderer (1) verwendet wird.

3. Verfahren nach Anspruch 1 oder 2, dadurch gekennzeichnet, dass als nicht oder schlecht fliessfähiges Pulver eine Mischung aus Formoterol (8) und Lactose (9) verwendet wird.

4. Verfahren nach Anspruch 3, dadurch gekennzeichnet, dass die Mischung im Mischungsverhältnis Formoterol zu Gesamtmischung von 1:10 bis 1:500 verwendet wird.

5. Verfahren nach Anspruch 3 oder 4, dadurch gekennzeichnet, dass Formoterol (8) mit einer mittleren Korngrösse von etwa 5 µm und Lactose (9) mit einer mittleren Korngrösse von 1µm bis zu etwa 50 µm, vorzugsweise mit einer mittleren Korngrösse von 1µm bis 10µm, verwendet wird.

6. Verfahren nach einem der Ansprüche 1 bis 5, dadurch gekennzeichnet, dass Körneragglomerate (10) mit einem mittleren Durchmesser von etwa 50 µm bis 2000 µm gebildet werden.

7. Vorrichtung zur Dosierung kleinster Mengen eines nicht oder schlecht fliessfähigen Pulvers (8,9) in ein Aufnahmegefäss (7), mit Dosiermitteln (3,4) zur Bemessung der Pulvermenge, mit Fördermitteln (13) zum Transport des Pulvers zu den Dosiermitteln (3,4) und mit Abfüllmitteln (5,6) zum Weitertransport der mit den Dosiermitteln (3,4) bemessenen Pulvermenge zum Aufnahmegefäss (7), dadurch gekennzeichnet, dass eine Vibrationseinrichtung (1) die nicht oder schlecht fliessfähigen feinen Körner (8,9) des Pulvers vor oder beim Transport zu den Dosiermitteln (3,4) mittels mechanischer Vibrationen zu fliessfähigen Körneragglomeraten (10) zusammenballt.

8. Vorrichtung nach Anspruch 7, dadurch gekennzeichnet, dass die Frequenz der Vibrationen vorzugsweise im Bereich um 100 Hz liegt.

9. Vorrichtung nach Anspruch 7 oder 8, dadurch gekennzeichnet, dass die Vibrationseinrichtung als Vibrationsförderer (1) ausgebildet ist und die fliessfähigen Körneragglomerate ( 10) zu den Dosiermitteln (3,4) führt.

10. Vorrichtung nach einem der Anprüche 7 bis 9, dadurch gekennzeichnet, dass die Dosiermittel eine von oben befüllbare Dosierkammer (31,32) und ein Auslassventil umfassen.

11. Vorrichtung nach Anspruch 10, dadurch gekennzeichnet, dass sie einen ersten Fülltrichter (2) umfasst, welcher so angeordnet ist, dass er die fliessfähigen Körneragglomerate (10) der Dosierkammer (31,32) direkt zuführt.

12. Vorrichtung nach Anspruch 10 oder 11, dadurch gekennzeichnet, dass die Dosierkammer durch eine durchgehende Oeffnung (31,32) in einer bewegbaren ersten Platte (3) und das Auslassventil durch eine ortsfeste zweite Platte (4) gebildet ist, die mit einer Oeffnung (41,42) versehen ist, wobei die Dosierkammer (32) in einer ersten Relativstellung der beiden Platten (3,4) durch die zweite Platte (4) verschlossen ist und in einer zweiten Relativstellung der beiden Platten (3,4) die Oeffnung (42) der zweiten Platte (4) mit der Oeffnung (32) der ersten Platte (3) fluchtet und dadurch die Dosierkammer (32) geöffnet ist.

13. Vorrichtung nach Anspruch 12, dadurch gekennzeichnet, dass Mittel (A) zum automatischen Hin- und Herbewegen der ersten Platte (3) zwischen den beiden Relativstellungen vorgesehen sind.

14. Vorrichtung nach Anspruch 12 oder 13, dadurch gekennzeichnet, dass die Abfüllmittel zum Weitertransport der mit den Dosiermitteln bemessenen Pulvermenge einen zweiten Fülltrichter (5) umfassen, der die durch die Oeffnung (41,42) der zweiten Platte (4) hindurchtretende Pulvermenge auffängt.

15. Vorrichtung nach Anspruch 14, dadurch gekennzeichnet, dass der zweite Fülltrichter (5) in ein Rohr (6) mündet, welches die vom Fülltrichter (5) aufgefangene Pulvermenge dem Aufnahmegefäss (7) zuführt.

16. Vorrichtung nach Anspruch 15, dadurch gekennzeichnet, dass die zweite Platte (4) mit dem Fülltrichter (5) baulich verbunden ist.

17. Vorrichtung nach einem der Ansprüche 7 bis 16, dadurch gekennzeichnet, dass die Dosiermittel und die Abfüllmittel als eine bauliche Einheit ausgebildet sind.

## Claims

1. A method of metering very small quantities of a non-flowable or poorly flowable fine-grained powder (8, 9), in which the powder is fed to a metering chamber (31, 32) and that chamber is emptied regularly, wherein, before the powder is fed to the metering chamber (31, 32), the non-flowable or poorly flowable grains (8, 9) of the powder are agglomerated by mechanical vibrations to form flowable grain agglomerations (10).

2. A method according to claim 1, wherein a vibratory conveyor (1) is used for agglomerating the grains to form flowable grain agglomerations (10) and for subsequently feeding those agglomerations (10) to the metering chamber (31, 32).

3. A method according to claim 1 or claim 2, wherein the non-flowable or poorly flowable powder used is a mixture of formoterol (8) and lactose (9).

4. A method according to claim 3, wherein the mixture is used in a mixing ratio of formoterol to the total mixture of from 1:10 to 1:500.

5. A method according to claim 3 or claim 4, wherein formoterol (8) having an average grain size of approximately 5 µm and lactose (9) having an average grain size of from 1 µm to approximately 50 µm, preferably having an average grain size of from 1 µm to 10 µm, are used.

6. A method according to any one of claims 1 to 5, wherein grain agglomerations (10) having an average diameter of approximately from 50 µm to 2000 µm are formed.

7. An apparatus for metering very small quantities of a non-flowable or poorly flowable powder (8, 9) into a collecting vessel (7), having metering means (3, 4) for measuring out the quantity of powder, having conveyor means (13) for transporting the powder to the metering means (3, 4), and having filling means (5, 6) for transporting the quantity of powder measured out by the metering means (3, 4) to the collecting vessel (7), wherein a vibration device (1) causes the non-flowable or poorly flowable fine grains (8, 9) of the powder to agglomerate by means of mechanical vibrations to form flowable grain agglomerations (10) before or during transport to the metering means (3, 4).

8. An apparatus according to claim 7, wherein the frequency of the vibrations is preferably in the region of 100 Hz.

9. An apparatus according to claim 7 or claim 8, wherein the vibration device is in the form of a vibratory conveyor (1) and feeds the flowable grain agglomerations (10) to the metering means (3, 4).

10. An apparatus according to any one of claims 7 to 9, wherein the metering means comprise a metering chamber (31, 32) which can be filled from above, and an outlet valve.

11. An apparatus according to claim 10, which comprises a first filling funnel (2) which is so arranged that it feeds the flowable grain agglomerations (10) directly to the metering chamber (31, 32).

12. An apparatus according to claim 10 or claim 11, wherein the metering chamber is formed by a through-opening (31, 32) in a movable first plate (3), and the outlet valve is formed by a fixed second plate (4) which is provided with an opening (41, 42), wherein in a first relative position of the two plates (3, 4) the metering chamber (32) is closed by the second plate (4), and in a second relative position of the two plates (3, 4) the opening (42) in the second plate (4) is aligned with the opening (32) in the first plate (3) and the metering chamber (32) is thus open.

13. An apparatus according to claim 12, wherein means (A) are provided for moving the first plate (3) to and fro automatically between the two relative positions.

14. An apparatus according to claim 12 or claim 13, wherein the filling means for transporting the quantity of powder measured out by the metering means comprise a second filling funnel (5) which collects the powder passing through the opening (41, 42) in the second plate (4).

15. An apparatus according to claim 14, wherein the second filling funnel (5) ends in a pipe (6) which feeds the quantity of powder collected by the filling funnel (5) to the collecting vessel (7).

16. An apparatus according to claim 15, wherein the second plate (4) is structurally joined to the filling funnel (5).

17. An apparatus according to any one of claims 7 to 16, wherein the metering means and the filling means are constructed as a structural unit.

## Revendications

1. Procédé pour l'addition dosée de quantités minimes d'une poudre (8, 9) à grains fins dotée d'une fluidité nulle ou d'une mauvaise fluidité, dans lequel la poudre est amenée dans une chambre (31, 32) d'introduction dosée ("chambre de dosage") et dans lequel celle-ci est vidée régulièrement, caractérisé en ce qu'avant l'alimentation en poudre de la chambre (31, 32) de dosage, les grains (8, 9) dotés d'une fluidité nulle ou d'une mauvaise fluidité sont rassemblés en boules au moyen de vibrations mécaniques en formant des agglomérats (10) de grains dotés de fluidité.

2. Procédé selon la revendication 1, caractérisé en ce que, pour le rassemblement en boules d'agglomérats (10) de grains dotés de fluidité et pour le transport, qui suit, de ces agglomérats (10) jusque dans la chambre de dosage (31, 32), on utilise un alimentateur vibrant (1).

3. Procédé selon la revendication 1 ou 2, caractérisé en ce que l'on utilise, en tant que poudre dotée d'une fluidité nulle ou d'une mauvaise fluidité, un mélange de formotérol (8) et de lactose (9).

4. Procédé selon la revendication 3, caractérisé en ce que le mélange utilisé présente un rapport de mélangeage formotérol/mélange total compris entre 1 à 10 et 1 à 500.

5. Procédé selon la revendication 3 ou 4, caractérisé en ce que l'on utilise du formotérol (8) dont la grosseur moyenne des grains est d'environ 5 µm et du lactose (9) dont la grosseur moyenne des grains est de 1 µm à environ 50 µm, et dont la grosseur moyenne des grains est de préférence de 1 µm à 10 µm.

6. Procédé selon l'une des revendications 1 à 5, caractérisé en ce que les agglomérats (10) de grains qui sont formés ont un diamètre moyen d'environ 50 µm à 2 000 µm.

7. Dispositif pour l'addition ou introduction dosée de quantités minimes d'une poudre (8, 9) dotée d'une fluidité nulle ou d'une mauvaise fluidité dans un récipient (7), le dispositif comportant des moyens de fourniture dosée ou moyens de dosage (3, 4) destinés à la mesure de la quantité de poudre (à délivrer), des moyens de transport (13) destinés à transporter la poudre jusqu'aux moyens de dosage (3, 4) et des moyens de remplissage (5, 6) destinés à poursuivre le transport de la quantité de poudre mesurée à l'aide des moyens de dosage (3, 4) jusqu'au récipient (7), caractérisé en ce qu'un dispositif vibrant (1) rassemble en boules les grains fins (8, 9) de la poudre,dotés d'une fluidité nulle ou d'une mauvaise fluidité, avant le transport, ou lors du transport, jusqu'aux moyens de dosage (3, 4) à l'aide de vibrations mécaniques pour les transformer en agglomérats (10) de grains dotés d'une bonne fluidité.

8. Dispositif selon la revendication 7, caractérisé en ce que la fréquence de vibrations est de préférence de l'ordre de 100 Hz.

9. Dispositif selon la revendication 7 ou 8, caractérisé en ce que le dispositif de vibration a une conformation d'alimentateur vibrant (1) et conduit les agglomérats (10) de grains jusqu'aux moyens de dosage (3, 4).

10. Dispositif selon l'une des revendications 7 à 9, caractérisé en ce que les moyens de dosage comprennent une chambre de dosage (31, 32),susceptible d'être remplie à partir du haut, et une valve d'échappement.

11. Dispositif selon la revendication 10, caractérisé en ce qu'il comprend une première trémie (2) de remplissage, qui est disposée de façon telle qu'elle conduit directement les agglomérats (10) de grains dotés d'une bonne fluidité à la chambre de dosage (31, 32).

12. Dispositif selon la revendication 10 ou 11, caractérisé en ce que la chambre de dosage est formée par une ouverture traversante (31, 32) pratiquée dans une première plaque (3) mobile, et en ce que la valve d'échappement est formée par une deuxième plaque (4) fixe, qui est munie d'une ouverture (41, 42), la chambre de dosage (32) étant fermée par la deuxième plaque (4) dans le cas d'une première position relative des deux plaques et, l'ouverture (42) de la deuxième plaque (4) étant en alignement avec l'ouverture (32) de la première plaque, dans le cas d'une seconde position relative des deux plaques (3, 4), la chambre de dosage (32) se trouvant de ce fait ouverte.

13. Dispositif selon la revendication 12, caractérisé en ce que des moyens (A) sont prévus pour le déplacement automatique de la plaque dans un sens et dans l'autre entre les deux positions relatives.

14. Dispositif selon la revendication 12 ou 13, caractérisé en ce que les moyens de remplissage comprennent, en vue de la poursuite du transport de la quantité de poudre mesurée à l'aide des moyens de fourniture dosée, une deuxième trémie (5) de remplissage qui récupère la quantité de poudre passant par l'ouverture (41, 42) de la deuxième plaque (4).

15. Dispositif selon la revendication 14, caractérisé en ce que la deuxième trémie (5) de remplissage débouche dans un tube (6) qui conduit la quantité de poudre récupérée par la trémie (5) de remplissage jusqu'au récipient (7).

16. Dispositif selon la revendication 15, caractérisé en ce que la deuxième plaque (4) est reliée structurellement à la trémie (5) de remplissage.

17. Dispositif selon l'une des revendications 7 à 16, caractérisé en ce que les moyens de fourniture dosée et les moyens de remplissage forment une unité structurelle.
